# EUROPEAN PATENT APPLICATION

(11) **EP 4 804 232 A1**
(43) Date of publication of application: **09.09.2026**
(21) Application number: 26162106.4
(22) Date of filing: 03.03.2026
(51) Int. Cl.: H01J 49/00, G01N 33/68

(54) **TRIGGERING HIGH-RESOLUTION MASS SPECTROMETER ANALYSES OF ISOBARIC LABELED REPORTER IONS**

(30) Priority: 05.03.2025 US 202563767108 P
(71) Applicant: Thermo Finnigan LLC, San Jose, CA 95134 (US)
(72) Inventor: MCALISTER, Graeme, San Jose, 95134 (US)
(74) Representative: Boult Wade Tennant LLP

(57) **Abstract**

A method of mass analyzing analytes within a mixture of samples, wherein all analytes from each sample from which the mixture is derived comprise a same isotopic label and wherein a different unique isotopic label is associated with each respective sample, the method comprising: cleaving, within the mass spectrometer, a reporter ion species from ions of an analyte of the mixture, wherein the reporter ion species comprises all of the isotopic labels from each of the differently-labeled samples; mass analyzing the reporter ion species at a first mass-spectral resolution; comparing an observed pattern of intensities of the mass analyzed reporter ion species to a predetermined intensity pattern; and if the observed intensity pattern fails to match the predetermined intensity pattern, mass analyzing the reporter ion species at a second mass spectral resolution that is greater than the first resolution.

## Description

### TECHNICAL FIELD

The present application relates, in general, to mass spectrometers and mass spectrometry. More particularly, the present application relates to analysis of isotopically labeled samples and, still more particularly, to analysis of samples having isobaric isotopic labels.

### BACKGROUND

A mass spectrometer is a sensitive instrument that may be used to detect, identify, and/or quantify molecules based on their mass-to-charge ratio (*m*/*z*)*.* A mass spectrometer generally includes an ion source for generating ions from components included in the sample, a mass analyzer for separating the ions based on their *m*/*z,* and an ion detector for detecting the separated ions. The mass spectrometer may be connected to a computer-based software platform that uses data from the ion detector to construct a mass spectrum that shows a relative abundance of each of the detected ions as a function of *m*/*z.* The *m*/*z* of ions may be used to detect and quantify molecules in simple and complex mixtures. A separation device such as a liquid chromatograph (LC) or gas chromatograph (GC) may be coupled to the mass spectrometer (MS) in a combined system (e.g., an LC-MS or GC-MS system) to separate components included in the sample before the components are introduced to the mass spectrometer.

The selective detection and quantitation of a specific analyte of interest in a complex mixture is often very difficult, even with targeted acquisition. For example, in proteomics research, a peptide of interest may be included in a complex biological matrix composed of a mixture of tens of thousands of peptides with abundances spanning many orders of magnitude. Tandem mass spectrometry (MS/MS or MS2) may be used for the quantitation of a molecule in a complex mixture. For example, in a refined targeted acquisition technique known as internal standard triggered-parallel reaction monitoring (IS-PRM), a sample containing a peptide target may be spiked with a known amount of the corresponding internal standard (IS) (e.g., a synthetic peptide with the same amino acid sequence but containing heavy stable isotope(s)) and scanned by the instrument in a dual-mode combining single stage and tandem mass spectrometry. Detection of the internal standard triggers the mass spectrometer to monitor for the specific endogenous peptide target of interest.

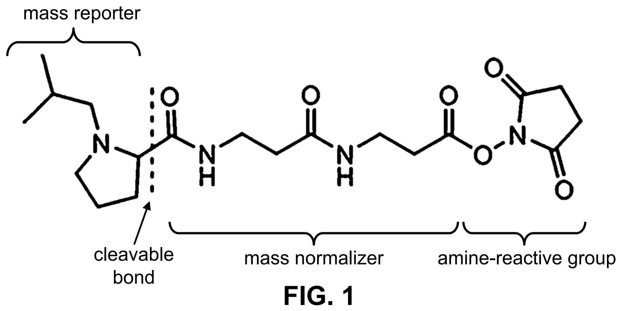

Multiplexing with the use of isobaric mass tags, such as Tandem Mass Tag^{®} (TMT^{®}) reagents (produced by Electrophoretics Limited and available from Thermo Fisher Scientific, Waltham, MA) and/or isobaric tags for relative and absolute quantitation (iTRAQ^{®}) (AB Sciex Pte. Ltd.), may increase sample throughput. Isobaric mass tags are compounds that react with and attach to analytes, such as peptides. As illustrated in FIG. 1, isobaric mass tags have a "reporter region", a "mass balance region", and a reactive group (e.g., an amine-reactive group, a cysteine-reactive group, or a carbonyl-reactive group). Versions of isobaric mass tags have been created that all have the same exact total mass of reporter region plus balance region, but the reporter region mass and the balance region mass for each version is different using various different combinations and positions of stable isotopes (e.g., ¹³C and ¹⁵N isotopes).

The use of isobaric mass tags is advantageous during proteomic studies since it enables a single mass analysis of a mixture of samples to replace an otherwise-required plurality of multiple mass analyses in which each sample would be analyzed individually. Multiple individual samples may be multiplexed by labeling analytes (e.g., peptides) in each sample with a different version of the isobaric mass tag, combining all the samples together, and analyzing the combined samples via LC-MS or GC-MS in one experiment. According to the isobaric labeling approach, the peptide or protein analytes of each sample are tagged by a respective isotopic label prior to creating the mixture. Although each tagged analyte gains mass by the tagging procedure, the mass gains are identical across all samples, because the any changes in mass created by isotopic substitution in the reporter region are counteracted by corresponding mass reductions in the "mass balance region". Accordingly, during detection (and possible isolation) of specific analyte ion species during the mass analysis, all similar analytes from different samples are detected and/or isolated simultaneously because they all comprise the same *m*/*z* value, regardless of provenance.

Cleavage of the bond identified by the dashed line in FIG. 1 liberates each reporter ion. An isobaric mass tag-based mass spectrometry experiment may thus determine a relative quantity of an analyte in each of multiple different samples. To this end, analytes in each sample, including any possible reference sample, are labeled with an isobaric mass tag. Most reporter ions have *m*/*z* values in the range of 126-135 Th. Labelling reagents are available that generate isobaric tags that require higher-energy collisional dissociation (HCD) to liberate the various mass reporter ions. Prior to the liberation of the mass reporter ions, the structure of a protein or peptide analyte of interest may be identified by standard MS/MS analysis using a lower-energy procedure, such as resonant excitation collisional dissociation to generate protein or peptide fragment ions having *m*/*z* values that may be input to a database search. Subsequently, the protein or peptide fragment ions may be subjected to MS3 analysis using HCD to cleave reporter ions. The relative intensity ratios of the various so liberated reporter ions at their various *m*/*z* values are indicative of the relative concentrations of the analytes across the various individual samples.

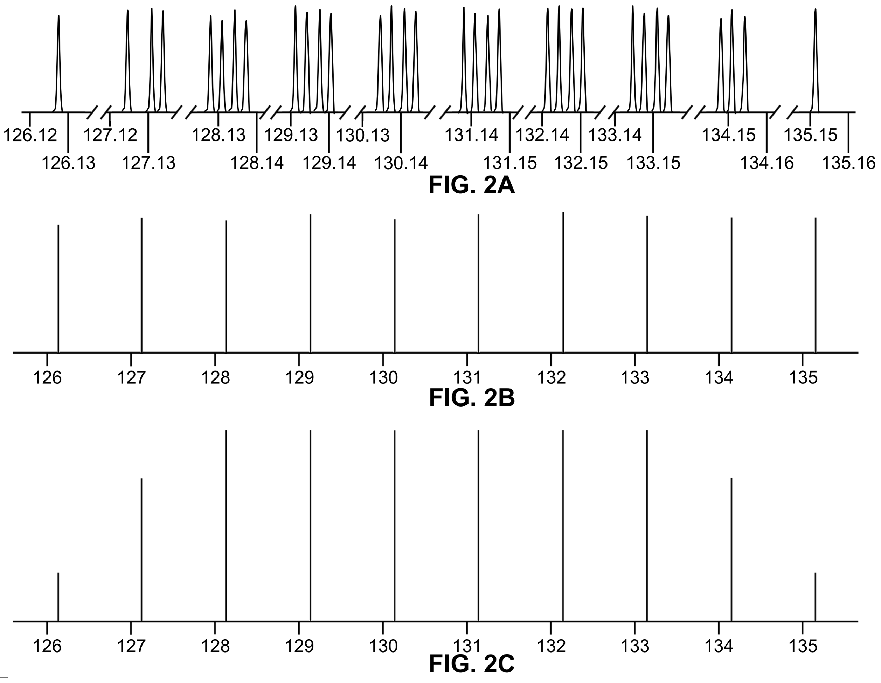

Recently, Thermo Fisher Scientific, of Waltham, Massachusetts, has introduced a new set of tandem mass tag (TMT) labeling that include deuterated species that differ by 3 mTh from the TMT labels previously available. The new TMT reagents approximately double the total number (i.e., from 18 to 32, presently) of reporter ions that may applied as labels to a suite of samples. For example, FIG. 2A shows the *m*/*z* values of the various reporter ions of the TMTpro^{™} 32-plex series of reagents (note the various breaks along the *m*/*z* axis). The signals of the multiple reporter ions are only resolvable using a high mass resolution mass analyzer, such as an Orbitrap^{™} mass analyzer. For comparison, FIG. 2B shows the forms of the various reporter-ion peaks when a standard resolution mass analyzer is employed (note that there are no breaks in this x-axis). Using a D20 Orbitrap operating at 4kV, to fully resolve all of these TMT reporter ions requires a 192 ms Orbitrap transient, which is twice as long as the transient needed to fully resolve the prior 18-plex set of TMT reagents. Obviously, this longer transient will slow down the MSn acquisition rate and limit the LC-MS sampling depth of mass analyzers when the full set of labels are applied to thirty-two different samples. The methods described here are intended to mitigate the impact of these longer transients by limiting the number of acquisitions in which it is necessary use these slower high-resolution mass spectrum acquisitions.

### BRIEF SUMMARY

According to a first aspect of the present disclosure, there is provided a method of mass analyzing analytes within a mixture of samples, wherein all analytes from each sample from which the mixture is derived comprise a same isotopic label and wherein a different unique isotopic label is associated with each respective sample, the method comprising:
cleaving, within the mass spectrometer, a reporter ion species from the ions of an analyte of the mixture, wherein the cleaved reporter ion species comprises all of the isotopic labels from each of the differently labeled samples;
mass analyzing the reporter ion species at a first mass-to-charge (*m*/*z*) resolution;
comparing an observed pattern of intensities versus mass of the mass analyzed reporter ion species to a predetermined intensity versus mass pattern; and
if the observed intensity pattern fails to match, within a tolerance, the predetermined intensity pattern, mass analyzing the reporter ion species at a second *m*/*z* resolution that is greater than the first *m*/*z* resolution.

According to the first aspect of the present disclosure, the distribution of an analyte among the samples may be determined from the mass analysis of the set of reporter ion species at the second *m*/*z* resolution. In various embodiments, the mass analyzing of the set of reporter ion species at the second *m*/*z* resolution requires a longer period of time than the mass analyzing of the set of reporter ion species at the first *m*/*z* resolution. In various embodiments, the mass spectrometer system comprises a first mass analyzer and a second mass analyzer, wherein the mass analyzing of the set of reporter ion species at the first *m*/*z* resolution is performed by the first mass analyzer and the mass analyzing of the set of reporter ion species at the second *m*/*z* resolution is performed by the second mass analyzer. In various embodiments, the mass-to-charge difference between at least some of the reporter ions may be less than 0.01 Th.

According to some embodiments, the method according to the first aspect may further include:
performing a tandem mass analysis of each analyte introduced into the mass spectrometer; and
identifying the structure and/or composition of the analyte based on the tandem mass analysis.
In such embodiments, each isotopic label may comprise a respective reporter-ion portion and a respective mass normalizer region, whereby the sum of the masses of the reporter-ion and mass normalizer regions is the same for all of the isotopic labels.

### BRIEF DESCRIPTION OF THE DRAWINGS

FIG. 1 is a depiction of the general chemical structure of various known tandem mass tag moieties;
FIG. 2A is a set of mass spectra of reporter ions of a set of 32 different mass tags;
FIG. 2B is a depiction of the form of mass spectral peaks of the reporter ions of FIG. 2A as would be observed during analysis by a standard-resolution mass spectrometer system;
FIG. 2C is an example of a predicted intensity pattern of reporter-ion relative intensities as may be observed in a low-resolution mass spectrum of a mixture of separately-isotopically-labeled samples in which the signals of individual reporter ions remain unresolved;
FIG. 3 is a functional diagram of an illustrative LC-MS system;
FIG. 4 is a functional diagram of an illustrative implementation of a mass spectrometer system;
FIG. 5 is a flow diagram of a method of mass spectrometry of isobaric labeled samples in accordance with the present teachings;
FIG 6 is a schematic depiction of a first exemplary mass spectrometer system on which methods of the present teachings may be practiced;
FIG. 7 is a schematic depiction of a second exemplary mass spectrometer system on which methods of the present teachings may be practiced; and
FIG. 8 is a functional diagram of an illustrative MS control system.

### DETAILED DESCRIPTION

As used in this application and in the claims, the singular forms "a", "an", and "the" include the plural forms unless the context clearly dictates otherwise. Additionally, the term "includes" means "comprises". Further, the term "coupled" does not exclude the presence of intermediate elements between the coupled items.

The systems, apparatuses, and methods described herein should not be construed as limiting in any way. Instead, the present disclosure is directed toward all novel and non-obvious features and aspects of the various disclosed embodiments, alone and in various combinations and sub-combinations with one another. The disclosed systems, methods, and apparatuses are not limited to any specific aspect or feature or combinations thereof, nor do the disclosed systems, methods, and apparatuses require that any one or more specific advantages be present or problems be solved. Any theories of operation are to facilitate explanation, but the disclosed systems, methods, and apparatuses are not limited to such theories of operation.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed systems, methods, and apparatuses can be used in conjunction with other systems, methods, and apparatuses. Additionally, the description sometimes uses terms like "produce" and "provide" to describe the disclosed methods. These terms are high-level abstractions of the actual operations that are performed. The actual operations that correspond to these terms will vary depending on the particular implementation and are readily discernible by one or ordinary skill in the art.

In some examples, values, procedures, or apparatuses are referred to as "lowest", "best", "minimum", "greater", "less than", "equal to" or the like. It will be appreciated that such descriptions are intended to indicate that a selection among many used functional alternatives can be made, and such selections need not be better, smaller, or otherwise preferable to other selections.

Multiplexed mass spectrometry for quantitation is often performed with a combined separation-mass spectrometry system, such as an LC-MS system. Accordingly, an LCMS system will now be described. The described LC-MS system is illustrative and not limiting. The methods and systems described herein may operate as part of or in conjunction with the LC-MS system described herein and/or with any other suitable separation-mass spectrometry system, such as a high-performance liquid chromatography-mass spectrometry (HPLC-MS) system, a gas chromatography-mass spectrometry (GC-MS) system, or a capillary electrophoresis-mass spectrometry (CEMS) system. The methods and systems described herein may also operate in conjunction with any other continuous flow sample source, such as a flow-injection mass spectrometry system (FI-MS) in which analytes are injected into a mobile phase (without separation in a column) and enter the mass spectrometer with time-dependent variations in intensity (e.g., Gaussian-like peaks). The method and systems described herein may also operate on a continuous flow sample source that provides a steady and consistent amount of sample, such that the sample measured by the mass spectrometer does not depend upon time.

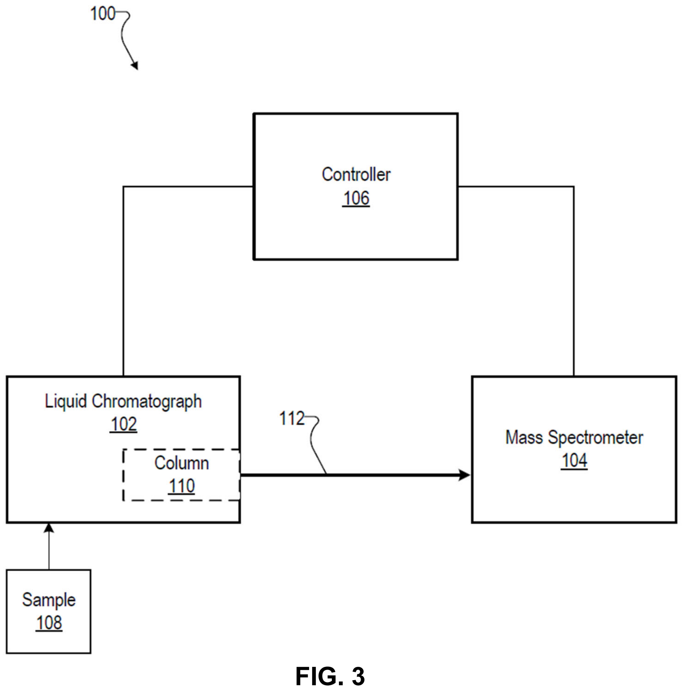

FIG. 3 shows a functional diagram of an illustrative LC-MS system **100.** LCMS system **100** includes a liquid chromatograph **102,** a mass spectrometer system **104,** and a controller **106.** Liquid chromatograph **102** is configured to separate, over time, analytes within a sample **108** that is injected into liquid chromatograph **102.** Sample **108** may include, for example, chemical analytes (e.g., molecules, ions, etc.) and/or biological analytes (e.g., metabolites, proteins, peptides, lipids, etc.) for detection and analysis by LC-MS system **100.** Liquid chromatograph **102** may be implemented by any liquid chromatograph as may suit a particular implementation. In liquid chromatograph **102,** sample **108** is injected into a mobile phase (e.g., a solvent), which carries sample **108** through a column **110** containing a stationary phase (e.g., an adsorbent packing material). As the mobile phase passes through column **110,** analytes within sample **108** elute from column **110** at different times based on, for example, their size, affinity to the stationary phase, polarity, and/or hydrophobicity.

A detector (e.g., an ion detector component of mass spectrometer system 104, an ion-electron converter and electron multiplier, etc.) may measure the relative intensity of a signal modulated by separated analytes in eluate **112** from column **110.** Data generated by the detector may be represented as a chromatogram, which plots retention time on the x-axis and a signal representative of the relative intensity on the yaxis. The retention time of an analyte is generally measured as the period of time between injection of sample **108** into the mobile phase and the relative intensity peak maximum after chromatographic separation. In some examples, the relative intensity may be correlated to or representative of relative abundance of the separated analytes. Data generated by liquid chromatograph **102** is output to controller **106.**

In some cases, particularly in analyses of complex mixtures, multiple different analytes in sample **108** co-elute from column **110** at approximately the same time, and thus may have the same or similar retention times. As a result, determination of the relative intensity of the individual analytes within sample **108** requires further separation of signals attributable to the individual analytes. To this end, liquid chromatograph **102** directs analytes included in eluate **112** to mass spectrometer system **104** for further separation, identification, and/or quantification of the analytes.

Mass spectrometer system **104** produces ions from the analytes received from liquid chromatograph **102** and sorts or separates the produced ions based on *m*/*z* of the ions. Mass spectrometer system **104** may be implemented by a multi-stage mass spectrometer configured to perform multi-stage mass spectrometry (also denoted MSn where n is 2 or more) or a tandem mass spectrometer configured to perform tandem mass spectrometry (a form of multi-stage mass spectrometry denoted MS/MS or MS2 (where n is 2)). A detector in mass spectrometer system **104** measures the intensity of the signal produced by the ions. As used herein, "intensity" or "signal intensity" refers to the response of the detector and may represent absolute abundance, relative abundance, ion count, intensity, relative intensity, ion current, or any other suitable measure of ion detection. Data acquired by mass spectrometer system **104** is output to controller **106.** Data generated by the detector may be represented by mass spectra, which plot the intensity of the observed signal as a function of *m*/*z* of the detected ions.

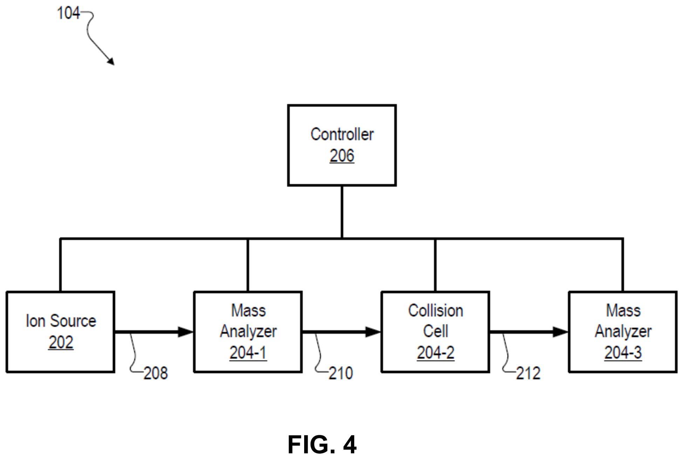

FIG. 4 shows a functional diagram of an illustrative implementation of mass spectrometer system **104.** Mass spectrometer system **104** includes an ion source **202,** a first mass analyzer **204-1,** a collision cell **204-2,** a second mass analyzer **204-3,** and a controller **206.** Mass spectrometer system **104** may further include any additional or alternative components not shown as may suit a particular implementation (e.g., ion optics, filters, ion stores, an autosampler, a detector, etc.).

Ion source **202** produces a stream **208** of ions from the analytes received from column **110** and deliver the ions to first mass analyzer **204-1.** Ion source **202** may use any suitable ionization technique, including without limitation electron ionization, chemical ionization, matrix assisted laser desorption/ionization, electrospray ionization, atmospheric pressure chemical ionization, atmospheric pressure photoionization, inductively coupled plasma, and the like. Ion source **202** may include various components for producing ions from analytes included in sample **108** and delivering the ions to first mass analyzer **204-1.**

First mass analyzer **204-1** receives ion stream **208,** isolates precursor ions of a selected *m*/*z* range, and delivers a beam **210** of the precursor ions to collision cell **204-2.** Collision cell **204-2** receives beam **210** of precursor ions and produces product ions (e.g., fragment ions) via controlled dissociation processes. Collision cell **204-2** directs a beam **212** of product ions to second mass analyzer **204-3.** Second mass analyzer **204-3** filters and/or performs a mass analysis of the product ions.

Mass analyzers **204-1** and **204-3** isolate or separate ions according to *m*/*z* of each of the ions. Mass analyzers **204-1** and **204-3** may be implemented by any suitable mass analyzer, such as a quadrupole mass filter, an ion trap (e.g., a three-dimensional quadrupole ion trap, a cylindrical ion trap, a linear quadrupole ion trap, a toroidal ion trap, etc.), a time-of-flight (TOF) mass analyzer, an electrostatic trap mass analyzer (e.g. an orbital electrostatic trap such as an Orbitrap mass analyzer, a Kingdon trap, etc.), a Fourier transform ion cyclotron resonance (FT-ICR) mass analyzer, and the like. Mass analyzers **204-1** and **204-3** need not be implemented by the same type of mass analyzer.

Collision cell **204-2** may be implemented by any suitable collision cell. As used herein, "collision cell" may encompass any structure or device configured to produce product ions via controlled dissociation processes and is not limited to devices employed for collisionally-activated dissociation. For example, collision cell **204-2** may be configured to fragment precursor ions using collision induced dissociation, electron transfer dissociation, electron capture dissociation, photo induced dissociation, surface induced dissociation, ion/molecule reactions, and the like.

An ion detector (not shown) detects ions at each of a variety of different *m*/*z* and responsively generates an electrical signal representative of ion intensity. The electrical signal is transmitted to controller **206** for processing, such as to construct a mass spectrum of the analyzed ions. For example, mass analyzer **204-3** may emit an emission beam of separated ions to the ion detector, which is configured to detect the ions in the emission beam and generate or provide data that can be used by controller **206** to construct a mass spectrum. The ion detector may be implemented by any suitable detection device, including without limitation an electron multiplier, a Faraday cup, and the like. In other examples, such as when second mass analyzer **204-3** is implemented by an orbital electrostatic trap mass analyzer, second mass analyzer **204-3** functions as both a mass analyzer and a detector.

Controller **206** is communicatively coupled with, and configured to control operations of, mass spectrometer system **104.** For example, controller **206** may be configured to control operation of various hardware components included in ion source **202** and/or mass analyzers **204-1** and **204-3.** To illustrate, controller **206** may be configured to control an accumulation time of ion source **202** and/or mass analyzers **204,** control an oscillatory voltage power supply and/or a DC power supply to supply an RF voltage and/or a DC voltage to mass analyzers **204,** adjust values of the RF voltage and DC voltage to select an effective *m*/*z* (including a mass tolerance window) for analysis, and adjust the sensitivity of the ion detector (e.g., by adjusting the detector gain).

Controller **206** may include any suitable hardware (e.g., a processor, circuitry, etc.) and/or software as may serve a particular implementation. While FIG. 4 shows that controller **206** is included in mass spectrometer system **104,** controller **206** may alternatively be implemented in whole or in part separately from mass spectrometer system **104,** such as by a computing device communicatively coupled to mass spectrometer system **104** by way of a wired connection (e.g., a cable) and/or a network (e.g., a local area network, a wireless network (e.g., Wi-Fi), a wide area network, the Internet, a cellular data network, etc.). In some examples, controller **206** is implemented in whole or in part by controller **106.**

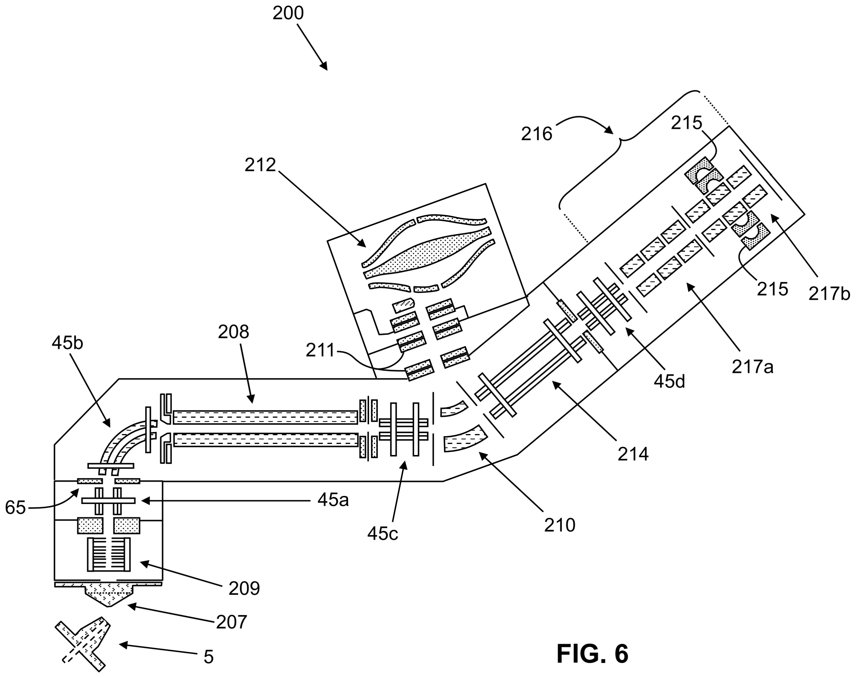

In the example of FIG. 4, mass spectrometer system **104** is tandem-in-space (e.g., has multiple mass analyzers) and has two stages for performing tandem mass spectrometry. However, mass spectrometer system **104** is not limited to this configuration but may have any other suitable configuration. For example, mass spectrometer system **104** may be tandem-in-time. Additionally or alternatively, mass spectrometer system **104** may be a multistage mass spectrometer and may have any suitable number of mass analyzers and stages (e.g., three or more) for performing multi-stage tandem mass spectrometry (e.g., MS/MS/MS). Examples of specific mass spectrometry systems that may employed are discussed in detail below in relation to FIGS. 6-7.

In typical, "real world", biological experiments, peptide abundances are quite consistent between samples. For example, most samples comprise various "housekeeping" proteins that do not vary a substantial amount between tissues or cell states. As such, the abundances of such proteins do not vary significantly between samples. Thus, when protein samples are tagged with isobaric isotopic labels, the ratios of reporter ions for the peptides generated by digestion of those proteins will be consistently uniform across most samples, assuming that the relative abundances of the various tagging moieties, at the time of applying the labels, are themselves essentially uniform, as shown in FIG. 2A. (If the relative abundances of the tagging moieties at the time of application are not uniform, then the relative abundances of reporter ions from these housekeeping proteins at the time of analysis will be non-uniform but will nonetheless be systematic.)

These presumed uniform reporter-ion intensities represent the basis for the formulation of a testable "null hypothesis" for quantitative measurements using isobaric labels. For example, for a fully resolved set of TMT reporter ions (FIG. 2A) and under the assumption of uniform application of tagging moieties (FIG. 2A), the null hypothesis presumes that, for each protein or peptide analyte that is detected by mass spectrometry, the abundance of said protein or peptide is uniform across all samples. Under this assumption, uniform reporter-ion intensities are expected across all of the TMT reporter ions (FIG. 2A). This statement ignores the effect of isotopic impurities, but those minor adjustments to the reporter ion intensities can be compensated for given the reagent specifications. In the case of unresolved TMT reporter ions, one can still expect a consistent set of TMT reporter ion intensities that are non-uniform but nonetheless follow a predictable intensity pattern, said pattern being the sum of all the unresolved peaks. In the case of FIG. 2C, the measured ratios would be 1:3:4:4:4:4:4:4:3:1 if, in each case, all of the fine isotopic differences of each set (i.e., corresponding to *m*/*z* ≈ 126 Th, 127 Th, ..., 135 Th) were to collapse down to a single respective peak. This particular example assumes that the 32 presently available different isotopologues are all present in a sample mixture in equal quantities, and that their individual mass spectra are unresolved, thereby yielding the 1:3:4:4:4:4:4:4:3:1 pattern. Any deviation from this expected pattern (or any other expected pattern, depending at least on the number of labels utilized), outside of a predetermined acceptable tolerance, would refute the null hypothesis and would indicate that one of the underlying TMT reporter ion intensities has deviated from the presumed 1:1 distribution. The deviation from the expected ratio may be the result of the presence of an analyte of interest that is either more abundant or less abundant in one or more samples that are used to create the mixture relative to the remaining samples. This increased or decreased abundance may possibly be the result of different treatments applied to the various samples from which the mixture is derived or, alternatively, different origins of the samples. The question of whether an observed intensity pattern matches a pre-determined intensity pattern within a certain acceptable tolerance may be decided by any suitable means of data comparison. As but one example, each of the observed pattern and the pre-determined pattern may be considered as a separate vector in *m*/*z* versus intensity space (or mass versus intensity space). With this calculation, the degree of matching of the two sets of data may be considered in terms of a difference vector or, alternatively, in terms of an angle between the two vectors.

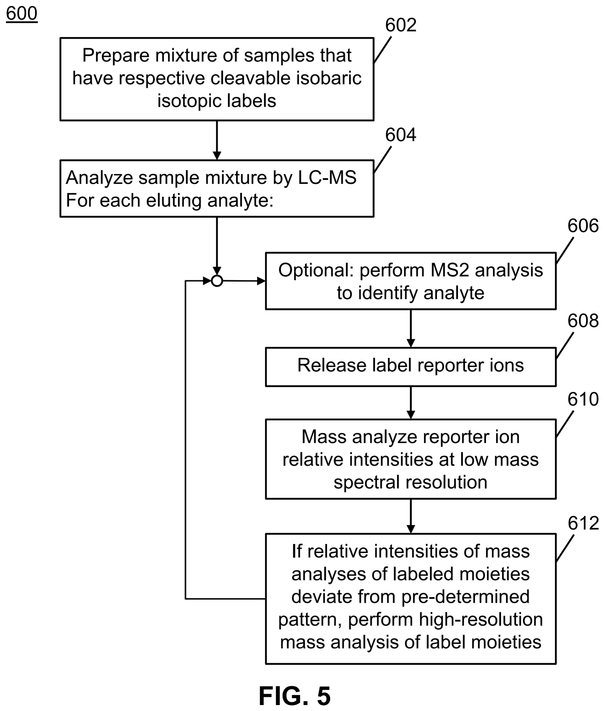

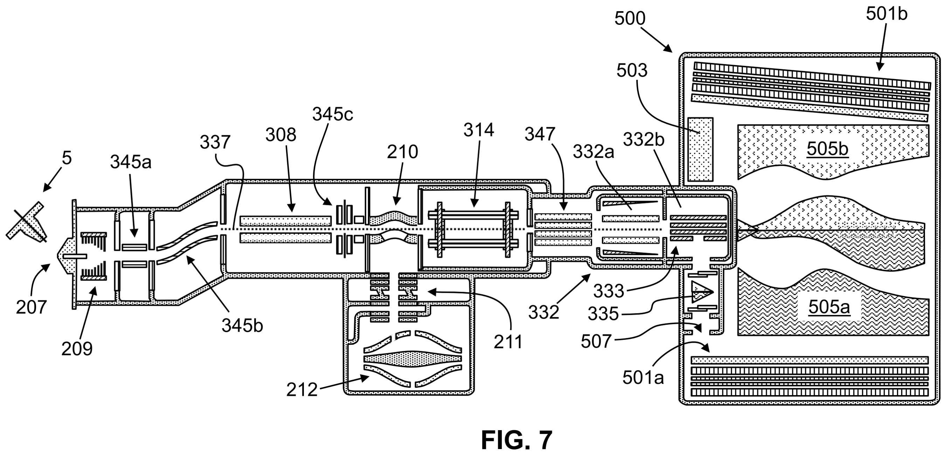

The above rationale indicates that, by real-time analysis of the TMT reporter ion ratios at standard resolution, it is possible to detect a deviation from the presumed null-hypothesis distribution, and accordingly instruct the mass spectrometer instrument to trigger a higher resolution quantitative scan that fully resolves the entire set of TMT reporter ions. FIG. 5 is a flow diagram of a method **600** that implements such a procedure. In step **602,** the proteins or peptides of each one of a plurality samples is tagged with a respective isobaric isotopic label and the samples are mixed, preferably in equal proportions, so as to create a sample mixture. In step **604,** the mixture is introduced into a liquid chromatography mass spectrometry (LC-MS) analytical system (e.g. system **100**) whereby the mixture is introduced into and fractionated by a column **110** of a chromatograph **102** and wherein each analyte is introduced into a mass spectrometer system (e.g., mass spectrometer system **104,** mass spectrometer system **200,** mass spectrometer system **300**) as it elutes from the chromatograph at its respective elution time. As each analyte is introduced into the mass spectrometer, it is ionized by an ion source (e.g., ion source **5,** as shown in FIG. 6 and FIG 7), thereby forming a stream of ions of the analyte that are routed through and processed by various components of the mass spectrometer system.

In step **606** of the method **600,** either the introduced ions or else ions generated by processing (e.g., fragmentation) of the introduced ions are directed into a mass analyzer (e.g., Orbitrap^{™} mass analyzer **212,** linear ion trap mass analyzer **217b,** Astral mass analyzer **500**) for detection of the *m*/*z* value(s) of the originally introduced ions or fragment ions. This step may optionally include identifying the analyte through recognition of diagnostic *m*/*z* values recorded from MS2 analysis. In step **608,** the various reporter ions are cleaved from their associated mass-balancing chemical structures and released as free ions by HCD fragmentation.

In step **612,** the released reporter ions are detected at standard or low mass spectral resolution and the relative intensities of the reporter ions are compared to a pre-determined intensity pattern that is consistent with the "null hypothesis" described above which presumes that the abundance of the analyte protein or peptide is uniform across all samples. The pre-determined intensity pattern depends on the actual relative abundance, taken across all samples of the mixture, of either the analyte itself or of a precursor molecule from which the analyte was derived. In certain cases, the pre-determined intensity pattern may also depend on the relative abundances, across samples, of the applied isotopic tag moieties at the time of labeling and/or the mixing ratios of the samples. In some cases, the "pre-determined" pattern may consist of a "rolling-average" of all the low-resolution intensity patterns previously measured within the LC-MS experiment. As such, the "pre-determined" pattern may be created prior to the performing of a particular MS2 analysis in question but not prior to the LC-MS experiment itself. If, in step 612, it is found that the low-resolution intensity pattern does not conform to the predetermined "null hypothesis" intensity pattern, then a high-resolution mass analysis of the reporter-ion region (e.g., the *m*/*z* range 126 Th to 135 Th) to resolve the separate signals of each of the reporter ion moieties.

FIGS. 6 and 7 are more-detailed schematic depictions of exemplary mass spectrometer systems which conform to the general system architecture shown in FIG. 4. Both the mass spectrometer system **200** (FIG. 6) and the mass spectrometer system **300** (FIG. 7), which are commercially available from Thermo Fisher Scientific of Waltham Massachusetts USA, are hybrid systems that utilize more than one mass analyzer. Specifically, the mass spectrometer system **200** includes an ion trap mass analyzer **216** as well as an Orbitrap^{™} analyzer **212,** which is a type of electrostatic trap mass analyzer. In operation of the mass spectrometer system **200,** an electrospray ion source **5** provides ions of a sample to be analyzed to an ion inlet aperture **207,** at which the ions enter into a first intermediate-vacuum chamber. After entry, the ions are captured and focused into a tight beam by a stacked-ring ion guide **209** or by an ion funnel at the same position. A first multipole ion guide **45** transfers the beam into downstream chambers of the mass spectrometer, where the various chambers are differentially pumped such that each succeeding chamber is maintained at a lower pressure than the preceding chamber. The second multipole ion guide **45b** is curved so as to cause neutral molecules to be separated from the main ion beam. Specifically, the neutral molecules follow a straight-line path whereas the ions of interest are caused to bend around a ninety-degree turn by a drag field applied to the ion guide **45b,** thereby producing the separation.

A quadrupole mass filter **208** of the mass spectrometer system **200** is used in its conventional sense as a tunable mass filter so as to pass ions only within a selected narrow mass-to-charge (*m*/*z*) range. A subsequent third ion guide **45c** delivers the filtered ions to a curved quadrupole ion trap ("C-trap") component **210.** The C-trap **210** is able to transfer ions along a pathway between the quadrupole mass filter **208** and the ion trap mass analyzer **216.** The C-trap **210** also has the capability to temporarily collect and store a population of ions and then deliver the ions, as a pulse or packet, into the Orbitrap^{™} mass analyzer **212.** The transfer of packets of ions is controlled by the application of electrical potential differences between the C-trap **210** and a set of injection electrodes **211** disposed between the C-trap **210** and the Orbitrap^{™} mass analyzer **212.** The curvature of the C-trap is designed such that the population of ions is spatially focused so as to match the angular acceptance of an entrance aperture of the Orbitrap^{™} mass analyzer **212.**

Multipole ion guide **214** (also referred to as "ion-routing multipole") and ion guide **45d** serve to guide ions between the C-trap **210** and the ion trap mass analyzer **216.** The multipole ion guide **214** provides temporary ion storage and can also serve as a fragmentation cell. Various gate electrodes along the pathway between the C-trap **210** and the ion trap mass analyzer **216** are controllable such that ions may be transferred in either direction (e.g., from multipole ion guide either towards ion trap mass analyzer **216** or towards C-trap **210**), depending upon the sequence of ion processing steps required in any particular analysis method.

The ion trap mass analyzer **216** is a dual-pressure linear ion trap (i.e., a two-dimensional trap) comprising a high-pressure linear trap cell **217a** and a low-pressure linear trap cell **217b,** the two cells being positioned adjacent to one another separated by a plate lens having a small aperture that permits ion transfer between the two cells and that presents a pumping restriction and allows different pressures to be maintained in the two traps. The environment of the high-pressure cell **217a** favors ion cooling, but also favors ion fragmentation under controlled conditions by either collision-induced dissociation or electron transfer dissociation or ion-ion reactions such as proton-transfer reactions. The low-pressure cell **217b** is a mass analyzer as the lower pressure of cell **217b** favors analytical scanning with high resolving power and mass accuracy. The low-pressure cell includes a dual-dynode ion detector **215.** The architecture of the mass spectrometer system **200** provides for efficient operation since different packets of ions may be either processed or analyzed in synchronicity in two or more of the quadrupole mass filter **208,** the C-trap **210,** the Orbitrap **212,** the ion-routing multipole **214,** the high-pressure linear ion trap **217a** and the low-pressure linear ion trap **217b.**

FIG. 7 is a detailed schematic depiction of another exemplary mass spectrometer system **300** which is commercially available from Thermo Fisher Scientific of Waltham Massachusetts USA under the name Orbitrap^{™} Astral^{™}. In similarity to the mass spectrometer system **200** (FIG 6), the mass spectrometer system **300** includes an ion source **5,** an ion inlet aperture **207,** and a plurality of vacuum chambers. Also included are an Orbitrap^{™} mass analyzer and its associated C-trap **210** and injection optics **211.** In further similarity to the mass spectrometer system **200** the mass spectrometer system **300** further includes a first multipole ion guide **345a,** a curved second multipole ion guide **345b,** a quadrupole mass filter **308,** a third multipole ion guide **345c,** and an ion routing multipole **314.** These further components perform functions that are analogous to the functions of, respectively, the first multipole ion guide **45a,** the second multipole ion guide **45b,** the quadrupole mass filter **208** and the third multipole ion guide **45c** of the mass spectrometer system **200.** Nonetheless, the components of the system **300** are not necessarily identical in structure to the corresponding components of the mass spectrometer system **200.**

The mass spectrometer system **300** (FIG. 7) significantly differs from the mass spectrometer system **200** of FIG. 6 in that the dual-pressure ion trap mass analyzer **216** of the latter system, is replaced, in the system **300,** by an Astral^{™} time-of-flight mass analyzer **500** that yields significantly higher resolution than the ion trap mass analyzer. Details of the mass analyzer **500** are described in US Patent No. 9,136,102, the disclosure of which is hereby incorporated herein by reference in its entirety. Details of the Orbitrap^{™} and Astral^{™} mass analyzer combination are described in Stewart, Hamish I., Dmitry Grinfeld, Anastassios Giannakopulos, Johannes Petzoldt, Toby Shanley, Matthew Garland, Eduard Denisov et al. "Parallelized acquisition of orbitrap and astral analyzers enables high-throughput quantitative analysis." Analytical chemistry 95, no. 42 (2023): 15656-15664, which is also hereby incorporated herein in its entirety.

The introduction of ions into the mass analyzer **500,** after passage of the ions through the ion routing multipole **314** and an octupole ion guide **347,** is performed by an ion processor **332** that comprises both a high-pressure chamber **332a** and a low-pressure chamber **332b.** The high-pressure chamber **332a** comprises a multipole ion trap within which ions may be fragmented for purposes of MS/MS analyses. The low pressure chamber **332a** comprises a set **333** of repeller and extractor electrodes that inject packets of ions into an ion inlet **507** of the mass analyzer **500** along a direction that is essentially orthogonal to the direction of the general trajectory **337** of ions through the system components that are upstream of the mass analyzer **500.** Ion lenses **335** focus and shape beams of ions prior to their entry into the mass analyzer **500.**

The mass analyzer **500** includes a first set of ion mirrors **501a** and a second set of ion mirrors **501b** that are non-parallel to the first ion mirrors **501a.** After injection into the mass analyzer **500,** ions repeatedly oscillate between the mirrors **501a-501b** while, at the same time, slowly drifting parallel to the direction in which the mirrors converge, this drift being caused by the initial inclination of the ion trajectories. The convergence of the ion mirrors causes the drift to decelerate over the course of the first 12-13 oscillations between the ion mirrors. A first set **505a** of ion foil electrodes is disposed to one side of the plane of the oscillations of the ions and a second set **505b** of ion foil electrodes is disposed at the opposite side of the plane of oscillations. The shapes of the ion foil electrodes **505a, 505b,** when provided with appropriate electrical potentials, serve to maintain the correct trajectories of the ions between the ion mirrors **501a, 501b.**

Eventually, the drift of the ions is reversed by the tilt of ion mirrors **501b** as well as by refraction on the ion foil such that the ions drift back in the direction of the spatial divergence of the ion mirrors. During the course of this reverse drift, the ions undergo another 12-13 oscillations between the ion mirrors such that the ions' total path length between the mirrors is greater than 30 meters. This long drift length produces high-resolution spatial separation of ions in accordance with their respective *m*/*z* values. The spatially separated packets of ions are detected by a high-dynamic-range detector **503.** Like the mass spectrometer system **200** (FIG. 6), the architecture of the mass spectrometer system **300** provides for efficient operation since different packets of ions may be either processed or analyzed in synchronicity in two or more of the quadrupole mass filter **208,** the C-trap **210,** the Orbitrap **212,** the ion-routing multipole **214,** the high-pressure compartment **332a** of the ion processor **332** and the Astral^{™} mass analyzer **500.**

Returning, once again, to the method **600** of FIG. 5 as well as to the descriptions of the mass spectrometer systems **200** (FIG. 6) and **300** (FIG. 7) it may be seen that the provision of more than one mass analyzer within a single mass spectrometer system facilitates parallelization of tasks wherein a first mass analyzer is utilized for lower-resolution MS1, MS2 and semi-quantitative reporter-ion "survey" data acquisitions and a second mass-analyzer is utilized for quantitatively-accurate high-resolution reporter-ion measurements. The actual assignments of these tasks to specific mass analyzers is quite flexible. For example, the lower-resolution scans could be shorter-transient Orbitrap^{™} mass analyzer acquisitions, Astral^{™} mass acquisitions, or even ion trap scans using ion-trap mass analyzer **217b.** While the higher-resolution scans would most likely be longer Orbitrap^{™} acquisitions, they could also be multiple-pass Astral^{™} spectra. Also, while this method was conceived to limit the impact of the new 32-plex TMT reagents, the same approach could be used for the currently shipping 18-plex TMT reagents.

Although the present discussion has focused on isobaric labeling of samples using Tandem mass Tags (TMT), the mass spectrometric methods described herein are also applicable to the isotopic labeling technique known as "Isobaric Tags for Relative and Absolute Quantitation" (iTRAQ) which is based on the covalent labeling of the N-terminus and side chain amines of peptides from protein digestions with tags of varying mass. Likewise, the methods described herein are further applicable to the laboratory procedure known as "Stable Isotope Labeling with Amino Acids in Cell culture" (SILAC) in which separate cell populations are grown, under differing laboratory conditions or treatments, using growth media containing isotopically labeled amino acids. Each cell population is grown using amino acids that have a respective isotopic label that is different from all of the isotopic labels that are applied to the growth of the other cell populations. Analytes from all samples are then combined into a mixture. These analytes may comprise the native proteins of the cell populations or, alternatively, may comprise a suite of peptides generated by digestion of the proteins. The analytes of the mixture are mass analyzed using various mass spectrometry techniques that include detection of the distribution(s) of isotopic labels among the analytes. Because the different labels correspond to different cell growth conditions or treatments, the effects of these conditions or treatments may be quantified.

Referring once again to FIG. 3, controller **106** is communicatively coupled with, and configured to control operations of, LC-MS system **100** (e.g., liquid chromatograph **102** and mass spectrometer system **104**). Controller **106** may include any suitable hardware (e.g., a processor, circuitry, etc.) and/or software configured to control operations of and/or interface with the various components of LC-MS system **100** (e.g., liquid chromatograph **102** or mass spectrometer system **104**).

Controller **106** and/or controller **206** may also include and/or provide a user interface configured to enable user interaction with LC-MS system **100** or mass spectrometer system **104.** The user may interact with controller **106** and/or controller **206** via the user interface by tactile, visual, auditory, and/or other sensory type communication. For example, the user interface may include a display device (e.g., liquid crystal display (LCD) display screen, a touch screen, etc.) for displaying information (e.g., mass spectra, notifications, etc.) to the user. The user interface may also include an input device (e.g., a keyboard, a mouse, a touchscreen device, etc.) that allows the user to provide input to controller **106** and/or controller **206.** In other examples, the display device and/or input device may be separate from, but communicatively coupled to, controller **106** and/or controller **206.** For instance, the display device and the input device may be included in a computer (e.g., a desktop computer, a laptop computer, etc.) communicatively connected to controller **106** and/or controller **206** by way of a wired connection (e.g., by one or more cables) and/or a wireless connection.

Controller **106** acquires data acquired over time by LC-MS system **100.** The data may include a series of mass spectra including intensity values of ions produced from the analytes of sample **108** as a function of *m*/*z* of the ions. The series of mass spectra may be represented in a three-dimensional map in which elution time (e.g., retention time) is plotted along an X-axis of the map, *m*/*z* is plotted along a Y-axis of the map, and intensity is plotted along a Z-axis of the map. Spectral features on the map (e.g., Z-axis peaks of intensity) represent detection by LC-MS system **100** of ions produced from various analytes included in sample **108.** The X-axis and Z-axis of the map may be used to generate an elution profile (e.g., a mass chromatogram) that plots detected intensity as a function of time for a selected *m*/*z.*

As used herein, a "selected *m*/*z"* refers to a specific *m*/*z,* with or without a mass tolerance window (e.g., +/- 0.5 Th), or a narrow range of *m*/*z* (e.g., an isolation window with a width or range such as 20 Th, 10 Th, 4 Th, 3 Th, etc.). In an MS2 or MSn analysis, such as a data dependent MS2 analysis, a selected reaction monitoring (SRM) analysis, a multiple reaction monitoring (MRM) analysis, or a parallel reaction monitoring (PRM) analysis, the selected *m*/*z* corresponds to the *m*/*z* of the precursor ion that is isolated and subsequently fragmented to generate reporter ions for relative quantitation.

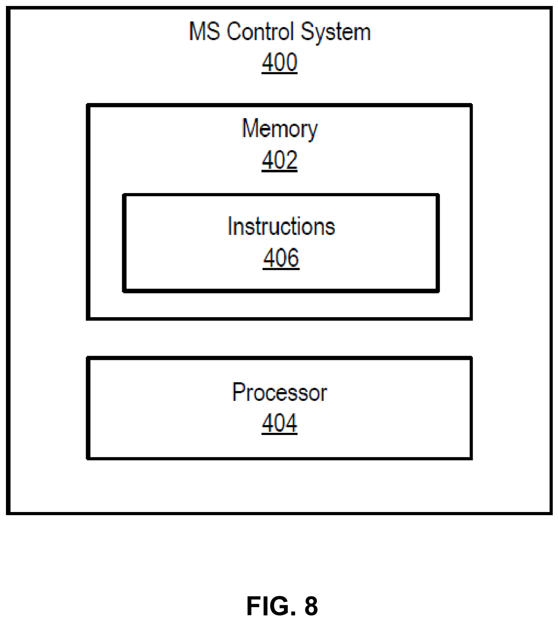

FIG. 8 shows a functional diagram of an illustrative MS control system **400** ("system 400"). System **400** may be implemented entirely or in part by an MS system, such as LC-MS system **100** (e.g., by controller **106** and/or controller **206**). Alternatively, system **400** may be implemented separately from the MS system (e.g., a remote computing system or server separate from but communicatively coupled to controller **106** and/or controller **206** of LC-MS system **100**). System **400** may include, without limitation, a memory **402** and a processor **404** selectively and communicatively coupled to one another. Memory **402** and processor **404** may each include or be implemented by hardware and/or software components (e.g., processors, memories, communication interfaces, instructions stored in memory for execution by the processors, etc.). Memory **402** and processor **404** may be distributed between multiple devices and/or multiple locations as may serve a particular implementation.

Memory **402** may maintain (e.g., store) executable data used by processor **404** to perform any of the operations described herein. For example, memory **402** may store instructions **406** that may be executed by processor **404** to perform any of the operations described herein. Instructions **406** may be implemented by any suitable application, software, code, and/or other executable data instance. Memory **402** may also maintain any data acquired, received, generated, managed, used, and/or transmitted by processor **404.** For example, memory **402** may maintain LC-MS data.

Processor **404** is configured to perform (e.g., execute instructions **406** stored in memory **402** to perform) various processing operations described herein. It will be recognized that the operations and examples described herein are merely illustrative of the many different types of operations that may be performed by processor **404.** In the description herein, any references to operations performed by system **400** may be understood to be performed by processor **404** of system **400.** Furthermore, in the description herein, any operations performed by system **400** may be understood to include system **400** directing, commanding, or instructing another system or device to perform the operations.

As used herein, an "acquisition" refers to a mass analysis performed at a discrete point in time to acquire a single mass spectrum, wherein a selected *m*/*z* is isolated and fragmented to generate an MS2 or MSn type acquisition. It will be recognized that, in some embodiments, the acquisition is based upon a data-dependent analysis, while in other embodiments it may be a targeted MS2 or data-independent analysis. In other embodiments, there may be multiple rounds of fragmentation and *m*/*z* selection, sometimes involving the simultaneous selection of multiple *m*/*z* values, all for the sake of performing a higher order MSn acquisition.

In the preceding description, various embodiments have been described. For purposes of explanation, specific configurations and details have been set forth in order to provide a thorough understanding of the embodiments. However, it will also be apparent to one skilled in the art that the embodiments may be practiced without the specific details. Furthermore, well-known features may have been omitted or simplified in order not to obscure the embodiment being described.

Some embodiments of the present disclosure include a system including one or more data processors and/or logic circuits. In some embodiments, the system includes a non-transitory computer readable storage medium containing instructions which, when executed on the one or more data processors, cause the one or more data processors to perform part or all of one or more methods and/or part or all of one or more processes and workflows disclosed herein. Some embodiments of the present disclosure include a computer-program product tangibly embodied in a non-transitory machine-readable storage medium, including instructions configured to cause one or more data processors to perform part or all of one or more methods and/or part or all of one or more processes disclosed herein.

The terms and expressions which have been employed are used as terms of description and not of limitation, and there is no intention in the use of such terms and expressions of excluding any equivalents of the features shown and described or portions thereof, but it is recognized that various modifications are possible within the scope of the claims. Thus, it should be understood that although the present disclosure includes specific embodiments and optional features, modification and variation of the concepts herein disclosed may be resorted to by those skilled in the art, and that such modifications and variations are considered to be within the scope of the appended claims.

## Claims

1. A method of mass analyzing analytes within a mixture of samples, wherein all analytes from each sample from which the mixture is derived comprise a same isotopic label and wherein a different unique isotopic label is associated with each respective sample, the method comprising:
cleaving, within the mass spectrometer system, a reporter ion species from the ions of an analyte of the mixture, wherein the reporter ion species comprises all of the isotopic labels from each of the differently labeled samples;
mass analyzing the reporter ion species at a first mass-to-charge (*m*/*z*) resolution;
comparing an observed pattern of intensities versus mass of the mass analyzed reporter ion species to a predetermined intensity versus mass pattern; and
if the observed intensity pattern fails to match, within a tolerance, the predetermined intensity pattern, mass analyzing the set of reporter ion species at a second *m*/*z* resolution that is greater than the first *m*/*z* resolution.

2. A method as recited in claim 1, wherein the distribution of the analyte among the samples is determined from the mass analysis of the set of reporter ion species at the second *m*/*z* resolution.

3. A method as recited in any previous claim, wherein the mass analyzing of the set of reporter ion species at the second *m*/*z* resolution is performed for a longer period of time than the mass analyzing of the set of reporter ion species at the first *m*/*z* resolution.

4. A method as recited in any previous claim, wherein the mass spectrometer system comprises a first mass analyzer and a second mass analyzer and wherein the mass analyzing of the set of reporter ion species at the first *m*/*z* resolution is performed by the first mass analyzer and the mass analyzing of the set of reporter ion species at the second *m*/*z* resolution is performed by the second mass analyzer.

5. A method as recited in any previous claim, wherein the mass-to-charge difference between at least some of the reporter ions is less than 0.01 Th.

6. A method as recited in any previous claim, further comprising:
performing a tandem mass analysis of each analyte introduced into the mass spectrometer; and
identifying the structure and/or composition of the analyte based on the tandem mass analysis.

7. A method as recited in any previous claim, wherein each isotopic label comprises a respective reporter-ion portion and a respective mass normalizer region, whereby the sum of the masses of the reporter-ion and mass normalizer regions is the same for all of the isotopic labels.

8. A method as recited in any previous claim wherein the predetermined intensity versus mass pattern is determined as a rolling average of previously-measured reporter-ion intensity patterns obtained using the first mass-to-charge resolution.

9. A liquid chromatography and mass spectrometry (LCMS) system comprising:
a liquid chromatograph configured to receive a mixture of samples, wherein analytes from each respective sample from which the mixture is derived comprise a same isotopic label and wherein a different unique isotopic label is associated with each respective sample;
a mass spectrometer system comprising at least one mass spectrometer; and
a controller electrically coupled to the liquid chromatograph and the mass spectrometer and comprising non-transient computer-readable instructions operable to:
cause the liquid chromatograph to fractionate the mixture and to provide, to the mass spectrometer system, a fraction of the mixture comprising an analyte of the sample, whereby the mass spectrometer system generates ions of the provided analyte;
cause the mass spectrometer system to cleave a plurality of reporter ion species from the ions of the provided analyte, wherein the reporter ion species comprise all of the isotopic labels from each of the differently labeled samples;
cause the mass spectrometer system to mass analyze the reporter ion species at a first mass-to-charge (*m*/*z*) resolution;
compare an observed pattern of intensities versus mass of the mass analyzed reporter ion species to a predetermined intensity versus mass pattern; and
if the observed intensity pattern fails to match the predetermined intensity pattern, within a tolerance, cause the mass spectrometer system to mass analyze the set of reporter ion species at a second *m*/*z* resolution that is greater than the first *m*/*z* resolution.

10. The LCMS system of claim 9, wherein the non-transient computer-readable instructions are further operable to:
determine a distribution of the analyte among the samples based on the mass analysis of the set of reporter ion species at the second *m*/*z* resolution.

11. The LCMS system of either one of claims 9-10, wherein the mass spectrometer system comprises:
a first mass analyzer; and
a second mass analyzer,
wherein the mass analyzing of the set of reporter ion species at the first *m*/*z* resolution is performed by the first mass analyzer and the mass analyzing of the set of reporter ion species at the second *m*/*z* resolution is performed by the second mass analyzer.

12. The LCMS system of any one of claims 9-11, wherein the mass-to-charge difference between at least some of the reporter ions is less than 0.01 Th.

13. The LCMS system of any one of claims 9-12, wherein the non-transient computer-readable instructions are further operable to:
cause the mass spectrometer system to perform a tandem mass analysis of the sample analyte; and
identify the structure and/or composition of the analyte based on the tandem mass analysis.

14. A computer program product tangibly embodied on a computer-readable medium comprising computer-readable instructions that are operable to:
cause a liquid chromatograph to fractionate a mixture of samples, wherein analytes from each sample from which the mixture is derived comprise a same isotopic label and wherein a different unique isotopic label is associated with each respective sample;
cause the liquid chromatograph to provide, to a mass spectrometer system, a fraction of the mixture comprising an analyte of the sample mixture, whereby the mass spectrometer system generates ions of the provided analyte;
cause the mass spectrometer system to cleave a set of reporter ion species from the ions of the provided analyte, wherein the reporter ion species comprise all of the isotopic labels from each of the differently labeled samples;
cause the mass spectrometer system to mass analyze the plurality of reporter ion species at a first mass-to-charge (*m*/*z*) resolution;
compare an observed pattern of intensities versus mass of the mass analyzed reporter ion species to a predetermined intensity versus mass pattern; and
if the observed intensity pattern fails to match, within a tolerance, the predetermined intensity pattern, cause the mass spectrometer system to mass analyze the plurality of reporter ion species at a second *m*/*z* resolution that is greater than the first *m*/*z* resolution.

15. The computer program product of claim 14, wherein the computer program product is further operable to determine the distribution of the analyte among the samples based on the mass analysis of the set of reporter ion species at the second *m*/*z* resolution.
